Europäisches Patentamt

European Patent Office

Office européen des brevets

(11) Publication number: **0 293 070**
A1

# EUROPEAN PATENT APPLICATION

(21) Application number: 88302859.9

(22) Date of filing: 30.03.88

(51) Int. Cl.⁴: **A61K 9/50** , **A61K 9/54**

| | |
|---|---|
| The title of the invention has been amended (Guidelines for Examination in the EPO, A-III, 7.3). | (71) Applicant: STRATHCLYDE CHEMICAL CO. LTD. 26 Muriel Street Barrhead Glasgow G78 1QH Scotland(GB) |
| (30) Priority: 01.04.87 GB 8707740 | (72) Inventor: Bruce, William Raymond 69 London Road Kilmarnock Scotland, KA3 7AH(GB) |
| (43) Date of publication of application: 30.11.88 Bulletin 88/48 | |
| | (74) Representative: Jones, Andrée Zena et al CRUIKSHANK & FAIRWEATHER 19 Royal Exchange Square Glasgow, G1 3AE Scotland(GB) |
| (84) Designated Contracting States: BE DE ES FR IT NL | |

(54) Oral composition for administering an active ingredient to an animal.

(57) The invention discloses an oral composition for treating infections in animals, for example, game birds and racing pigeons. A physiologically acceptable carrier such as quartz or limestone grit is coated either with a water-insoluble coating, say, tallow, containing an active ingredient or with a water-soluble layer containing a similar type of ingredient and further provided with an outer layer of a water-insoluble material to protect the soluble coating. Suitable active ingredients may be fenbendazole or levamisole hydrochloride.

EP 0 293 070 A1

# COMPOSITION

The present invention relates to compositions and in particular to oral compositions suitable for use in administering medicaments or nutrients to animals.

The administration of medicaments or nutrients to animals can involve considerable problems and/or logistical problems especially where relatively large numbers are involved in free-range conditions. Particular examples that may be mentioned are in the treatment of birds such as game birds or racing pigeons with anthelmintics in order to combat parasite infections, e.g. worm infections, such as trichostrongylus or Capillaria, tick-borne infections, trichomonas or coccidiosis. Game birds such as grouse normally eat only heather and grit, which they require to aid their digestion. Thus in practice in the case of game birds, these require to be caught and individually dosed orally or parenterally with the required medication, e.g. an anthelmintic drug.

The present invention provides an oral composition suitable for use in administering an active ingredient to an animal, comprising an oral composition suitable for use in administering an active ingredient to an animal, comprising a physiologically acceptable, orally ingestible carrier having a least one coating that contains said active ingredient, said coating having a form and composition such as to permit absorption of said active ingredient by said animal, wherein an outer coating of said carrier is water-insoluble.

In a further aspect the present invention provides a method of administering medicaments or nutrients to animals comprising the steps of providing an oral composition of the invention and supplying it to the animals and allowing them to ingest it.

Thus a composition of the present invention can be distributed to animals in the open air for administering any desired substance even in inclement weather conditions without the risk of most of the active ingredient being washed away by rain.

Various coating materials may be used in accordance with the present invention. In general though there is conveniently used a fat or relatively high melting point oil, usually an animal fat or a saturated fat or oil having a melting point above normal ambient temperature, say, at least 35° C. Specific coating materials that may be mentioned include palm oil fatty acid, hardened palm kernel oil and tallow.

Such coatings are themselves water-insoluble and therefore protect the active ingredient from the effects of the weather. However, where the active ingredient is contained in a first coating of water-soluble material such as agar, a second water-insoluble coating may be applied to protect the ingredient-containing layer. It will be understood that the melting point of the material of first coating must be higher than that of the outer coating and that the temperature at which the outer coating is applied is arranged at a level between the melting points of the materials of the two coatings.

The particular coating material to be used in any given case will depend on various factors including inter alia the thermal tolerance of the active ingredient. Thus for example in the case of levamisole hydrochloride which melts above 61° C and becomes denatured, it is therefore necessary to use coating material that has a melting point below 61° C so that it can be readily liquified without risk of damage to the active ingredient when this is mixed with the liquified coating material.

Any solid, physiologically acceptable, orally ingestible carrier may be used in a composition of the present invention depending on various factors such as the age and species of the animal and acceptability of the composition to the animal. Thus for example in the case of grouse or pigeons where quartz or limestone grit is a normal part of their intake under conventional rearing conditions, the carrier is conveniently in the form of quartz or limestone grit.

The coating material containing the active ingredient may be applied to the carrier by any suitable process for the coating of a solid carrier. In general though, the coating is liquified, e.g. melted by raising its temperature to above its melting point, applied to the carrier in any suitable manner e.g. by spraying, pouring or mixing, using a suitable mixing apparatus of, for example, the change-pan or barrel mixer type, and then solidified, e.g. by cooling and allowing the temperature of the coating to fall below its melting point.

In order to avoid the particles sticking together, they are desirably kept separate and/or mixing thereof is continued until the coating has solidified.

The thickness and amount of coating relative to the particle size may be varied within the relatively wide limit, according to a number of factors such as the animal species and the dozage requirements of the active ingredient. In general though the coating will be limited to a relatively thin layer from 2¹/2 to 4% by weight in order to minimize loss of coating, especially where this is of a generally non-rigid nature such as a soft fat, by mechanical displacement and transfer to the ground or other extraneous nature.

Various active ingredients may be employed in the compositions such as dietary supplements, e.g. minerals, trace elements, vitamins and the like or other nutrient materials, and medicaments of various sorts.

Medicaments that may be mentioned include anthelmintics, such as in the first example below, levamisole hydrochloride.

If desired other ingredients such as for example flavourings and/or scent, which are attractive to the target animal, may be incorporated in the coating in order to maximise uptake of the composition. Thus for example, in the case of a composition for grouse there could be incorporated a flavour such as oil of aniseed. In addition other material such as for example, antioxidants may advantageously be incorporated in the coating.

Further preferred features and advantages of the invention will appear from the following detailed examples of the invention given by way of illustration only.

Example I

Levamisole hydrochloride (160g) was added to molten, anti-oxidised tallow fat (3kg) at a temperature of 58°C, and the mixture thoroughly stirred. Quartz grit (100kg) average particle size diameter 1-3mm placed in a barrel type open-top mixer. The molten tallow fat was then sprayed on to the grit over a period of one minute with continuous stirring of the grit. Stirring was then continued for a further period of 15 minutes until the tallow had completely solidified as a coating on the individual grit particles.

Example II

A 2% solution of agar was heated to 100°C and 100gm Fenbendazole mixed with 1kg of the melted agar solution. The mixture was sprayed onto 100kg of 1-3mm quartz grit to coat the particles which were agitated in a suitable mixer, in the present example a U-trough mixer. The coated grit was allowed to cool below 50°C to allow the agar coating to set. Molten anti-oxidised tallow at 60-80°C was then sprayed on the coated grit to form an outer coating on the grit particles and the tallow allowed to set into a substantially weather-impervious layer.

**Claims**

1. An oral composition suitable for use in administering an active ingredient to an animal, comprising a physiologically acceptable, orally ingestible carrier having at least one coating that contains said active ingredient, said coating having a form and composition such as to permit absorption of said active ingredient by said animal, wherein an outer coating of said carrier is water-insoluble.

2. A composition as claimed in claim 1, wherein the coating containing the active ingredient comprises the water-insoluble outer coating.

3. A composition as claimed in claim 1, wherein the coating containing the active ingredient is water-soluble and is surrounded by said outer, water-insoluble, coating.

4. A composition as claimed in either one of claims 2 and 3, wherein the water-insoluble coating is a fat or oil having a melting point above normal ambient temperature.

5. A composition as claimed in claim 4 wherein the water-insoluble coating is palm oil fatty acid, hardened palm kernel oil or tallow.

6. A composition as claimed in claim 3, wherein the water soluble coating is an agar solution.

7. A composition as claimed in any one of the preceding claims, wherein the active ingredient is fenbendazole.

8. A composition as claimed in any one of claims 1-6, wherein the active ingredient is Fenbendazole.

9. A composition as claimed in any one of preceding claims wherein additions of nutrients trace elements or vitamin are added to the coating.

10. A composition as claimed in any one of the preceding claims wherein the carrier is quartz grit.

11. A composition as claimed in any one of the preceding claims wherein the carrier is limestone grit.

12. A method of administering medicaments or nutrients to animals comprising the steps of providing an oral composition according to claim 1, supplying it to the animals and allowing them to ingest the same.

## DOCUMENTS CONSIDERED TO BE RELEVANT

| Category | Citation of document with indication, where appropriate, of relevant passages | Relevant to claim | CLASSIFICATION OF THE APPLICATION (Int. Cl. 4) |
|---|---|---|---|
| X | EP-A-0 210 540 (FUJISAWA PHARMACEUTICAL CO., LTD) * Column 3, line 5 - column 4, line 50; column 1, lines 4-11; figure 3 * | 1,3 | A 61 K 9/50<br>A 61 K 9/54 |
| Y | --- | 4,5 | |
| X | WO-A-8 403 623 (BLOCH) * Page 13, line 1 - page 14, line 5 * | 2 | |
| P,Y | US-A-4 675 236 (OHKAWARA et al.) * Column 3, line 41 - column 4, line 33 * | 4,5 | |
| A | EP-A-0 198 769 (FOREST LABORATORIES) * Column 3, line 35; examples 1-3 * | 6 | |
| A | DE-A-2 606 531 (HOFFMANN-LA ROCHE) * Page 4, line 1 - page 5, line 6 * | 7,8,12 | |
| A | DE-A-2 647 562 (HESSE & CO.) * Claims * | 9,10 | TECHNICAL FIELDS SEARCHED (Int. Cl.4) |
| A | GB-A- 601 469 (AMERICAN DAIRIES) * Claim 2 * | 11 | A 61 K<br>A 23 K |

The present search report has been drawn up for all claims

| Place of search | Date of completion of the search | Examiner |
|---|---|---|
| THE HAGUE | 04-08-1988 | GOETZ G. |

EPO FORM 1503 03.82 (P0401)